# EUROPEAN PATENT APPLICATION

(11) **EP 4 582 126 A1**
(43) Date of publication of application: **09.07.2025**
(21) Application number: 24217022.3
(22) Date of filing: 03.12.2024
(51) Int. Cl.: A61M 16/08, A61M 16/04, G01F 1/36, A61M 16/00

(54) **INTEGRATED BREATHING CIRCUIT**

(30) Priority: 05.01.2024 US 202418405636
(71) Applicant: Honeywell International Inc., Charlotte, NC 28202 (US)
(72) Inventor: PINKERTON, Adam Joseph, Charlotte, 28202 (US); HOOVER, William, Charlotte, 28202 (US); YEE, Daniel, Charlotte, 28202 (US); AVASTHI, Rahul, Charlotte, 28202 (US)
(74) Representative: Haseltine Lake Kempner LLP

(57) **Abstract**

A breathing system, comprising a breathing circuit comprising a plurality of breathing tubes; a flow sensor coupled to the breathing circuit, the flow sensor comprising a housing defining a flow channel and having a plurality of coupling members, wherein each coupling member of the plurality of coupling members defines an opening therethrough; and a suction tube assembly. A first set of coupling members of the plurality of coupling members is coupled to an end of the suction tube assembly via one or more connectors, one or more valves are disposed between the first set of coupling members and the suction tube assembly.

## Description

### TECHNICAL FIELD

The present application relates generally to a breathing circuit. More specifically, the present application relates generally to a breathing circuit having a flow sensor integrated within the breathing circuit.

### BACKGROUND

A breathing circuit may be configured to direct the flow of breathing gas to a patient. For example, a breathing circuit may be used in an intubation procedure to provide respiration assistance to a patient.

The inventors have identified numerous deficiencies and problems with breathing circuits. Through applied effort, ingenuity, and innovation, many of these identified deficiencies and problems have been solved by developing solutions that are structured in accordance with the embodiments of the present disclosure, many examples of which are described in detail herein.

### BRIEF SUMMARY

In accordance with one aspect of the present disclosure, a breathing system is provided. The example breathing system includes a breathing circuit comprising a plurality of breathing tubes; a flow sensor coupled to the breathing circuit, the flow sensor comprising a housing defining a flow channel and having a plurality of coupling members, wherein each coupling member of the plurality of coupling members defines an opening therethrough; and a suction tube assembly, wherein: a first set of coupling members of the plurality of coupling members is coupled to an end of the suction tube assembly via one or more connectors, and one or more valves are disposed between the first set of coupling members and the suction tube assembly.

In some embodiments, the housing comprises a first end coupled to a first breathing tube of the plurality of breathing tubes and an opposing second end coupled to a second breathing tube of the plurality of breathing tubes.

In some embodiments, the one or more connectors comprise one or more secure leak-proof connectors.

In some embodiments, the one or more valves are configured for regulating removal of contamination within the flow sensor via the suction tube assembly.

In some embodiments, the housing comprises a first housing and a second housing, wherein a first coupling member of the first set of coupling members extends from the first housing, and a second coupling member of the first set of coupling members extends from the second housing.

In some embodiments, each of the first coupling member and the second coupling member comprises a luer lock fitting.

In some embodiments, the first coupling member is coupled to a first suction tube of the suction tube assembly, such that a first suction pathway is defined, and the second coupling member is coupled to a second suction tube of the suction tube assembly, such that a second suction pathway is defined.

In some embodiments, a first valve of the one or more valves is disposed between the first coupling member and the first suction tube, and a second valve of the one or more valves is disposed between the second coupling member and the second suction tube.

In some embodiments, an end of a third suction tube of the suction tube assembly is coupled to the first suction tube and the second suction tube via a connector.

In some embodiments, an opposing end of the suction tube assembly is configured for being coupled to a suction device.

In accordance with one aspect of the present disclosure, a breathing system is provided. The breathing system, comprises a breathing circuit comprising a plurality of breathing tubes; a flow sensor coupled to the breathing circuit, the flow sensor comprising a housing defining a flow channel and having at least one reservoir and a plurality of coupling members, wherein each coupling member of the plurality of coupling members defines an opening therethrough; and a suction tube assembly, wherein: a first set of coupling members of the plurality of coupling members is coupled to an end of the suction tube assembly via one or more connectors, and one or more valves are disposed between the first set of coupling members and the suction tube assembly.

In some embodiments, the housing comprises a first end coupled to a first breathing tube of the plurality of breathing tubes and an opposing second end coupled to a second breathing tube of the plurality of breathing tubes.

In some embodiments, the one or more connectors comprise one or more secure leak-proof connectors.

In some embodiments, the one or more valves are configured for regulating removal of contamination within the flow sensor via the suction tube assembly.

In some embodiments, the housing comprises a first housing having a first reservoir and a second housing having a second reservoir.

In some embodiments, a first coupling member of the first set of coupling members extends from the first reservoir, and a second coupling member of the first set of coupling members extends from the second reservoir.

In some embodiments, each of the first coupling member and the second coupling member comprises a luer lock fitting.

In some embodiments, the first coupling member is coupled to a first suction tube of the suction tube assembly, such that a first suction pathway is defined, and the second coupling member is coupled to a second suction tube of the suction tube assembly, such that a second suction pathway is defined.

In some embodiments, a first valve of the one or more valves is disposed between the first coupling member and the first suction tube, and a second valve of the one or more valves is disposed between the second coupling member and the second suction tube.

In some embodiments, an opposing end of the suction tube assembly is configured for being coupled to a suction device.

The above summary is provided merely for purposes of summarizing some example embodiments to provide a basic understanding of some aspects of the present disclosure. Accordingly, it will be appreciated that the above-described embodiments are merely examples and should not be construed to narrow the scope or spirit of the present disclosure in any way. It will be appreciated that the scope of the present disclosure encompasses many potential embodiments in addition to those here summarized, some of which will be further described below. Other features, aspects, and advantages of the subject matter will become apparent from the description, the drawings, and the claims.

### BRIEF DESCRIPTION OF THE DRAWINGS

Having thus described certain example embodiments of the present disclosure in general terms above, non-limiting and non-exhaustive embodiments of the subject disclosure are described with reference to the following figures, which are not necessarily drawn to scale and wherein like reference numerals refer to like parts throughout the various views unless otherwise specified. The components illustrated in the figures may or may not be present in certain embodiments described herein. Some embodiments may include fewer (or more) components than those shown in the figures.
FIG. 1 schematically illustrates a portion of an example breathing system in accordance with at least one example embodiment of the present disclosure.
FIGS. 2A is a schematic illustration of an example flow sensor of an example breathing system in accordance with at least one example embodiment of the present disclosure.
FIG. 2B is a schematic illustration of an example flow sensor of an example breathing system in accordance with at least one example embodiment of the present disclosure.
FIG. 3 schematically illustrates a portion of an example breathing system showing a suction tube assembly in accordance with at least one example embodiment of the present disclosure.
FIG. 4 schematically illustrates a portion of a breathing system showing a suction tube assembly in accordance with at least one example embodiment of the present disclosure.
FIG. 5 schematically illustrates a portion of a breathing system having reservoirs in accordance with at least one example embodiment of the present disclosure.
FIG. 6 illustrates a flowchart depicting operations of an example process for removing contamination from a flow sensor of an example breathing system in accordance with at least one example embodiment of the present disclosure.
FIGS. 7A-7B provides an example simulation result plot.

### DETAILED DESCRIPTION

One or more embodiments are now more fully described with reference to the accompanying drawings, wherein like reference numerals are used to refer to like elements throughout and in which some, but not all embodiments of the inventions are shown. In the following description, for purposes of explanation, numerous specific details are set forth in order to provide a thorough understanding of the various embodiments. It is evident, however, that the various embodiments can be practiced without these specific details. It should be understood that some, but not all embodiments are shown and described herein. Indeed, the embodiments may be embodied in many different forms, and accordingly this disclosure should not be construed as limited to the embodiments set forth herein. Rather, these embodiments are provided so that this disclosure will satisfy applicable legal requirements.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

As used herein, the phrases "in one embodiment," "according to one embodiment," "in some embodiments," and the like generally mean that the particular feature, structure, or characteristic following the phrase may be included in at least one embodiment of the present disclosure, and may be included in more than one embodiment of the present disclosure (importantly, such phrases do not necessarily refer to the same embodiment).

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments, or it may be excluded.

As used herein, the term "example" or "exemplary" means serving as an example, instance, or illustration. Any aspect or design described herein as "exemplary" is not necessarily to be construed as preferred or advantageous over other aspects or designs. Rather, use of the word exemplary is intended to present concepts in a concrete fashion. In addition, while a particular feature may be disclosed with respect to only one of several implementations, such feature may be combined with one or more other features of the other implementations as may be desired and advantageous for any given or particular application. Furthermore, to the extent that the terms "includes" and "including" and variants thereof are used in either the detailed description or the claims, these terms are intended to be inclusive in a manner similar to the term "comprising."

As used herein, the term "or" is used in both the alternative and conjunctive sense, unless otherwise indicated. The terms "illustrative" and "example" are used to be examples with no indication of quality level. Terms such as "computing," "determining," "generating," and/or similar words are used herein interchangeably to refer to the creation, modification, or identification of data. Further, "based on," "based on in part on," "based at least on," "based upon," and/or similar words are used herein interchangeably in an open-ended manner such that they do not indicate being based only on or based solely on the referenced element or elements unless so indicated. Like numbers refer to like elements throughout.

As used herein, the terms "coupled," "fixed," "attached to," and the like refer to both direct coupling, fixing, or attaching, as well as indirect coupling, fixing, or attaching through one or more intermediate components or features, unless otherwise specified herein.

As used herein, terms of approximation, such as "approximately," "substantially," or "about," refer to being within manufacturing or engineering tolerances. For example, terms of approximation may refer to being withing a five percent margin of error.

A breathing circuit may be configured to direct the flow of breathing gas to a patient. For example, a breathing circuit may be used in an intubation procedure to provide respiration assistance to a patient. Breathing gas may be supplied to the patient via the breathing circuit and a flow sensor may be attached to the breathing circuit to measure the flow rate. As described above Applicant has identified various deficiencies associated with breathing circuits. For example, when a patient is invasively intubated, a tube is passed down through the patient's throat. Mucus and/or other substances may flow out of the tube into a flow sensor attached to the breathing circuit.

Moreover, in various applications, a humidifier may be leveraged to maintain air flowing through the breathing circuit at a certain temperature and humidity level. The humidified air may condense inside the flow sensor or breathing circuit due to temperature differential between ambient air flowing through the circuit. Contamination, such as mucus, condensation, and/or the like within the flow sensor may cause the flow sensor to fail or otherwise adversely impact the performance of the flow sensor. For example, operation of internal component(s) of the flow sensor may be impaired, resulting in inaccurate flow rate measurement and/or other parameters.

To remove the mucus (and/or other substance) and the condensation, existing solutions require a medical provider to take the breathing circuit apart and clean the flow sensor, which exposes the patient to environmental conditions, and also exposes the medical provider to the medical condition the patient may have. Generally, the medical provider has to use a separate component (e.g., detached from the breathing circuit) to clean out the flow sensor, re-attach the sensor to the breathing circuit, and re-attach the breathing circuit to the patient. Additionally, flooding of the flow sensor can lead to stoppage of therapy, increased operational effort for clinicians, and increase in waste due to discarding flooded flow sensors.

Embodiments of the present disclosure address the above-mentioned challenges and difficulties, as well as other challenges and difficulties associated with breathing circuits. Embodiments of the present disclosure provide a breathing system comprising a breathing circuit with a flow sensor integrated therein, that allows for removal of contamination (e.g., mucus, condensation, and/or the like) from the flow sensor without taking apart the breathing circuit. Moreover, embodiments of the present disclosure allow for removal of contamination from the sensor while the patient is using the breathing system.

Some embodiments of the present disclosure include a suction tube assembly attached to the flow sensor. The suction tube assembly may be configured for being coupled to a suction device (e.g., a suction machine) configured to suck out contamination from the flow sensor via the suction tube assembly. Some embodiments of the present disclosure include one or more reservoirs configured to allow for more contamination to be collected before the need to clean the sensor, which, in turn, increases the time to failure of the sensor. By providing for cleaning the flow sensor without taking the breathing circuit apart, embodiments of the present disclosure prevent exposure of the patient to adverse environment conditions, prevents exposure of medical providers to the medical conditions associated with a patient, increases the performance (e.g., time to failure) of the flow sensor, and reduces the amount of resources that would otherwise be utilized to take the breathing circuit apart and clean (or replace) the flow sensor.

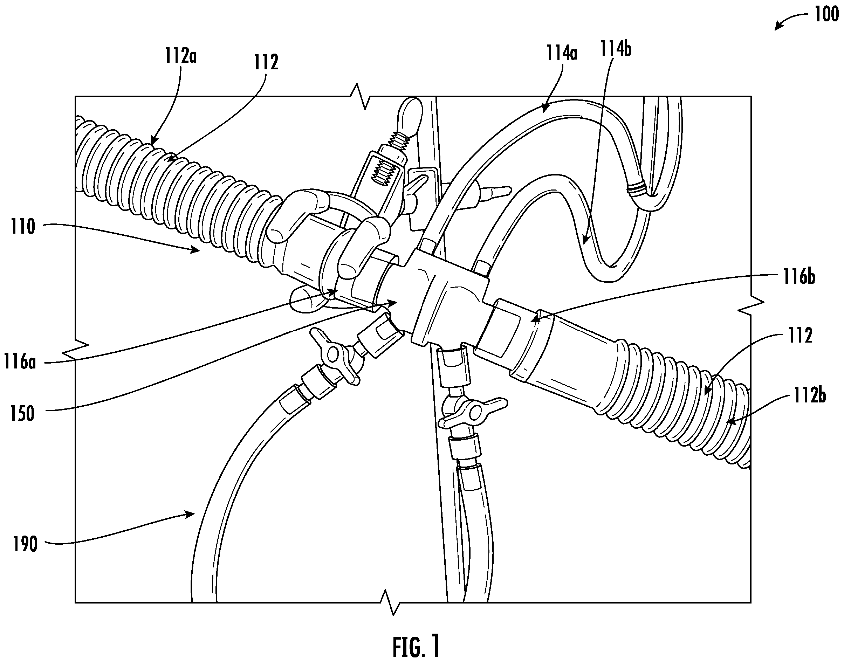

FIG. 1 is a schematic illustration of a portion of an example breathing system 100 in accordance with at least one example embodiment of the present disclosure. As shown in FIG. 1, the breathing system 100 may include a breathing circuit 110 and at least one flow sensor 150 integrated within the breathing circuit 110. For example, a flow sensor 150 may be coupled, directly or indirectly, between at least two components of the breathing circuit 110. In various embodiments, the breathing system 100 includes a suction tube assembly 190. The suction tube assembly 190 may be, directly or indirectly, coupled to the flow sensor 150. In various embodiments, the suction tube assembly 190 may be configured to facilitate removal of contamination from the flow sensor 150.

The breathing circuit 110 may include a plurality of breathing tubes 112 through which gas flows into and out of the flow sensor 150. The plurality of breathing tubes 112 may be made from a variety of materials. For example, the plurality of breathing tubes 112 may be made from, plastics, composites, and/or the like. In some embodiments, one or more of the plurality of breathing tubes 112 may be flexible. In some embodiments, one or more of the plurality of breathing tubes 112 may be rigid. In some embodiments, one or more of the plurality of breathing tubes 112 may be transparent. In some embodiments, one or more of the plurality of breathing tubes 112 may be translucent.

The breathing circuit 110 may include one or more connectors and/or one or more adapters. The one or more connectors and/or one or more adapters may be configured for coupling at least a portion of the plurality of breathing tubes 112 to one or more other components of the breathing system 100 and/or to one or more other devices. In various embodiments, and as shown in FIG. 1, an end of a first breathing tube 112a of the plurality of breathing tubes 112 may be coupled to the flow sensor 150 via a first adapter 116a, and an end of a second breathing tube 112b of the plurality of breathing tubes 112 may be coupled to the flow sensor 150 via a second adapter 116b. In various embodiments, one of the first breathing tube 112a or the second breathing tube 112b may be configured for coupling, directly or indirectly, to a ventilator device (e.g., via one or more connectors), while the other of the first breathing tube 112a or second breathing tube 112b may be configured for coupling, directly or indirectly, to a user (e.g., a medical patient). For example, an opposing end of the first breathing tube 112a may be configured for coupling, directly or indirectly, to a ventilator device, while an opposing end of the second breathing tube 112b may be configured for coupling, directly or indirectly, to a user. For example, an opposing end of the second breathing tube 112b may be configured for coupling, directly or indirectly, to a ventilator device, while an opposing end of the second breathing tube 112b may be configured for coupling, directly or indirectly, to a user. It would be appreciated that the breathing circuit may comprise different configurations in some examples. For example, the first and second breathing tubes may be coaxial (e.g., one breathing tube inside the other breathing tube) in some examples. As another example, the breathing circuit may comprise only a single tube in some examples.

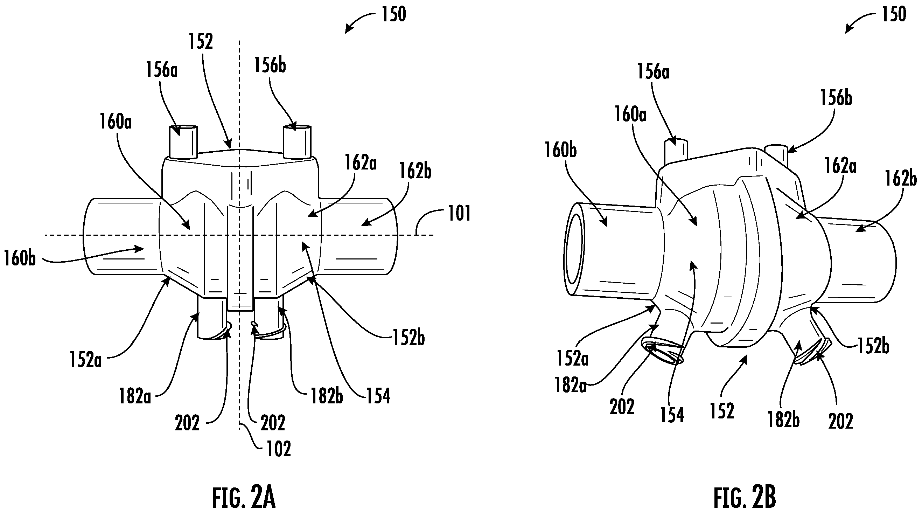

FIG. 2a is a schematic illustration of a flow sensor 150 in accordance with at least one example embodiment of the present disclosure. FIG. 2b is a schematic illustration of a flow sensor 150 in accordance with at least one example embodiment of the present disclosure. The flow sensor 150 may be a proximal flow sensor based on a differential pressure principle. For example, the flow sensor 150 may describe a passive flow differential pressure converter configured for measuring of gas flow (e.g., breathing gas flow). The flow sensor 150 may comprise a housing 152 defining a flow channel 154. In various embodiments, the flow channel 154 may define a flow path in a plane that is parallel (e.g., substantially parallel) to a horizontal-axis 101 defined by the housing 152. For example, gas may flow through the flow channel 154 from the first breathing tube 112a to the second breathing tube 112b (or vice versa) along the flow path.

In various embodiments, the housing 152 comprises a first housing 152a and a second housing 152b. The first housing 152a and the second housing 152b may be coupled together at a respective end portion of the first housing 152a and the second housing 152b. Each of the flow first housing and the second housing 152b may define a cavity therein. In various embodiments, the flow sensor 150 may be configured to determine the gas flow (e.g., flow rate) based on the differential pressure between the first housing 152a cavity and the second housing 152b cavity. For example, the differential pressure between the first housing 152a and the second housing 152b may be converted to a flow rate.

The first housing 152a may comprise a body 158a and the second housing 152b may comprise a body 158b. The body 158a of the first housing 152a may comprise a first body portion 160a and a second body portion 160b. The second body portion 160b may have a diameter that is smaller than the diameter of the first body portion 160a. The body 158b of the second housing 152b may comprise a first body portion 162a and second body portion 162b. The second body portion 162b may have a diameter that is smaller than the diameter of the first body portion 162a.

In various embodiments, the diameter of the first body portion 160a of the first housing 152a and the diameter of the first body portion 162a of the second housing 152b may be substantially the same. In various embodiments, the diameter of the first body portion 160a of the first housing 152a and the diameter of the first body portion 162a of the second housing 152b may be different. In various embodiments, the diameter of the second body portion 160b of the first housing 152a and the diameter of the second body portion 162b of the second housing 152b may be substantially the same. In various embodiments, the diameter of the second body portion 160b of the first housing 152a and the diameter of the second body portion 162b of the second housing 152b may be different.

In various embodiments, the housing 152 comprises a plurality of coupling members. Each coupling member of the plurality of coupling members may define an opening therethrough. In various embodiments, a set of one or more coupling members of the plurality of coupling member is configured to facilitate flow measurement. For example, the set of one or more coupling members may be configured for coupling the flow sensor 150 to sensor tube(s), where the sensor tube(s) may be coupled to one or more differential pressure measurement devices.

In various embodiments, each of the first housing 152a and the second housing 152b may comprise at least one coupling member. As shown in FIGS. 2a and 2b, the first housing 152a comprises a first coupling member 156a and the second housing 152b comprises a second coupling member 156b. In various embodiments, each of the first coupling member 156a and the second coupling member 156b may be configured for coupling the housing 152 to a sensor tube. In various embodiments, the first coupling member 156a may be coupled to a first sensor tube 114a such that the first coupling member 156a opens into the first sensor tube 114a. In various embodiments, the second coupling member 156b may be coupled to a second sensor tube 114b such that the second coupling member 156b opens into the second sensor tube 114b.

A respective opposing end of the first sensor tube 114a and the second sensor tube 114b may be configured for coupling, via one or more connectors, to a measuring unit comprising one or more differential pressure measurement devices (e.g., differential pressure measurement gauges). In some embodiments, the first coupling member 156a and/or the second coupling member 156b is positioned perpendicular (e.g., substantially perpendicular) with respect to the horizontal-axis 101. It would be appreciated that in some embodiments, the first coupling member 156a and/or the second coupling member 156b may be positioned at an angle with respect to the horizontal-axis 101 defined by the housing 152, where the noted angle is not a perpendicular.

In some embodiments, and as shown in FIGS. 2a and 2b, the first coupling member 156a may extend perpendicularly from the first body portion 160a of the first housing 152a and the second coupling member 156b may extend perpendicularly from the first body portion 162a of the second housing 152b. In various embodiments, the first coupling member 156a and the second coupling member 156b have substantially the same diameter. It would be appreciated that in some embodiments, the diameter of the first coupling member 156a and the diameter of the second coupling member 156b may be different.

The flow sensor 150 may include at least one flow resistance element (not shown) positioned within the housing 152. The at least one flow resistance element may be positioned within the flow sensor 150 in the area where the first housing 152a and second housing 152b meet. For example, a flow resistance element may be positioned substantially parallel to the vertical-axis defined by the housing 152 (thus, positioned substantially perpendicular to the horizontal axis defined by the housing 152, which corresponds to the flow path within the flow channel 154 of the housing 152. The flow resistance element may be leveraged to determine the difference in pressure upstream of the flow resistance element and downstream of the flow resistance element. For example, the flow resistance element may be leveraged to determine the difference in pressure within the first housing 152a and the second housing 152b. A flow resistance element may comprise a sheet, a film, or the like, and may be made from a variety of materials (e.g., plastic, composite, and/or the like). In some embodiments, a flow resistance element may be embodied as a flexible flap having a circular shape. In some embodiments, the flow resistance element may comprise a narrow slit. It would be appreciated that in other embodiments, the one or more flow resistance elements may comprise other configurations. For example, in some embodiments, a flow resistance element may have a non-circular shape profile.

A set of one or more coupling members of the plurality of coupling members of the housing 152 may be configured to facilitate removal of contamination (e.g., contamination, mucus, and/or the like) from the flow sensor 150. For example, the housing 152 may comprise a third coupling member 182a and a fourth coupling member 182b. The third coupling member 182a and the fourth coupling member 182b may each be configured to facilitate removal of contamination. As shown in FIGS. 2a and 2b, the third coupling member 182a and the fourth coupling member 182b may extend from the body 158a and the body 158b respectively in a direction opposite the direction in which the first coupling member 156a and second coupling member 156b extend from the body 158a and the body 158b, respectively. For example, the housing 152 may comprise a set of one or more coupling members configured to facilitate flow measurement, and a set of one or more coupling configured to facilitate removal of contamination from the flow sensor, where the sets of one or more coupling members are positioned on opposite sides of the housing 152.

The third coupling member 182a may extend from the first body portion 160a of the body 158a and the fourth coupling member 182b may extend from the first body portion 162a of the body 158b. As shown in FIG. 2a. in some embodiments, the third coupling member 182a may extend perpendicular from the body 158a (e.g., first body portion 160a thereof) and the fourth coupling member 182b may extend perpendicular from the body 158b (e.g., first body portion 162a thereof). As shown in FIG 2b. in some embodiments, the third coupling member 182a may extend at an angle that is not perpendicular with respect to the body 158a (e.g., first body portion 160a thereof) and the fourth coupling member 182b may extend at an angle that is not perpendicular with respect to the body 158b (e.g., first body portion 162a thereof). As shown in FIG. 2b, each of the third coupling member 182a and fourth coupling member 182b may extend from the body 158a and the body 158b respectively, such that the third coupling member 182a extends away with respect to the vertical axis 102 defined by the housing 152 and the fourth coupling member 182b extends away with respect to the vertical axis 102. It would be appreciated that in some embodiments, each of the third coupling member 182a and fourth coupling member 182b may extend from the body 158a and the body 158b respectively, such that the third coupling member 182a extends towards with respect to the vertical axis 102 defined by the housing 152 and the fourth coupling member 182b extends towards with respect to the vertical axis 102.

Each of the third coupling member 182a and the fourth coupling member 182b may define an opening therethrough (e.g., channel), such that contamination within the flow sensor 150 may be transmitted out of the flow sensor 150 via the third coupling member 182a and the fourth coupling member 182b. In various embodiments, the third coupling member 182a and the fourth coupling member 182b have substantially the same diameter. It would be appreciated that in some embodiments, the diameter of the third coupling member 182a and the diameter of the fourth coupling member 182b may be different.

As described above, in various embodiments, the breathing system 100 includes a suction tube assembly 190. Each of the third coupling member 182a and the fourth coupling member 182b may be configured for coupling, directly or indirectly the flow sensor 150 to the suction tube assembly 190. In various embodiments, the third coupling member 182a is coupled to a first suction tube of the suction tube assembly, such that a first suction pathway is defined, and the fourth coupling member 182b is coupled to a second suction tube of the suction tube assembly, such that a second suction pathway is defined.

In various embodiments, a secure leak-free connection configuration is leveraged to couple the third coupling member 182a and the fourth coupling member 182b to the suction tube assembly 190. In some embodiments, and as shown in FIGS. 2a and 2b, the third coupling member 182a and the fourth coupling member 182b may comprise a luer lock fitting configured to facilitate secure leak-proof connection between each of the third coupling member 182a and the fourth coupling member 182b and the suction tube assembly.

For example, and as shown in FIGS 2a and 2b, a portion of the outer surface of each of the third coupling member 182a and fourth coupling member 182b may comprise one or more ridges 202 along the outer surface configured to facilitate a secure leak-proof coupling. Each of the third coupling member 182a and the fourth coupling member 182b may comprise a male luer lock fitting configured to mate with a corresponding female luer lock fitting 308. It would be appreciated that in some embodiments, each of the third coupling member 182a and the fourth coupling member 182b may comprise a female luer lock fitting configured to mate with a corresponding male luer lock fitting. It would be appreciated that in some embodiments, the third coupling member 182a and the fourth coupling member 182b may comprise a secure leak proof coupling configuration that is different from a luer lock configuration.

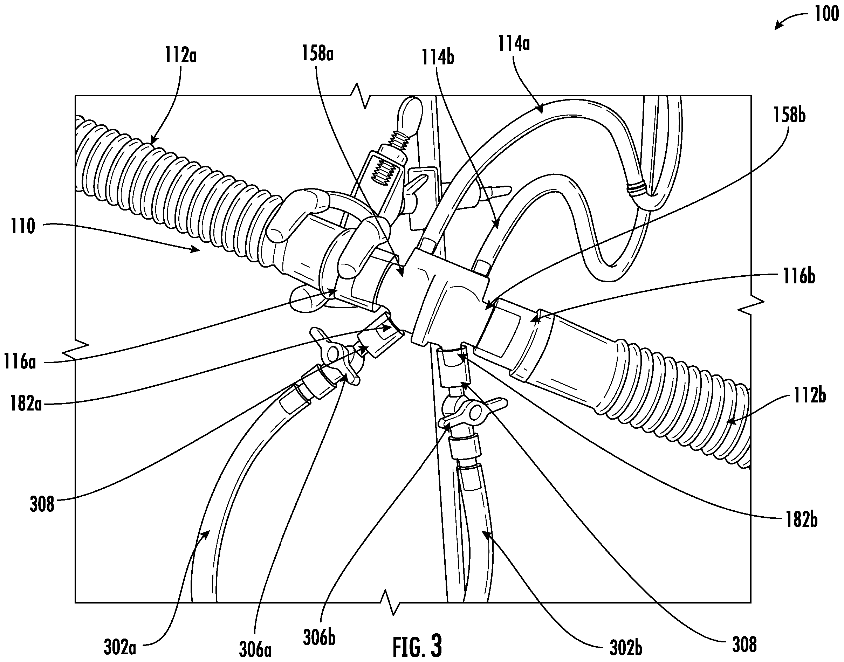

FIG. 3 schematically illustrates a portion of a breathing system 100 showing a suction tube assembly 190 in accordance with at least one example embodiment of the present disclosure. The suction tube assembly 190 may comprise one or more suction tubes. As shown in FIG. 3, an end of a first suction tube 302a of the suction tube assembly 190 may be coupled to the third coupling member 182a and an end of second suction tube 302b of the suction tube assembly 190 may be coupled to the fourth coupling member 182b. One or more connectors and/or one or more valves may be leveraged to couple the first suction tube 302a to the third coupling member 182a and the second suction tube 302b to the fourth coupling member 182b. In some embodiments, the one or more connectors comprise luer lock fittings, such as female luer lock fitting 308. In various embodiments, opposing ends of the first suction tube 302a and the second suction tube 302b may be configured for being coupled to a suction device. In some embodiments, a first valve 306a of the one or more valves is disposed between the first coupling member and the first suction tube, and a second valve 306b of the one or more valves is disposed between the second coupling member and the second suction tube.

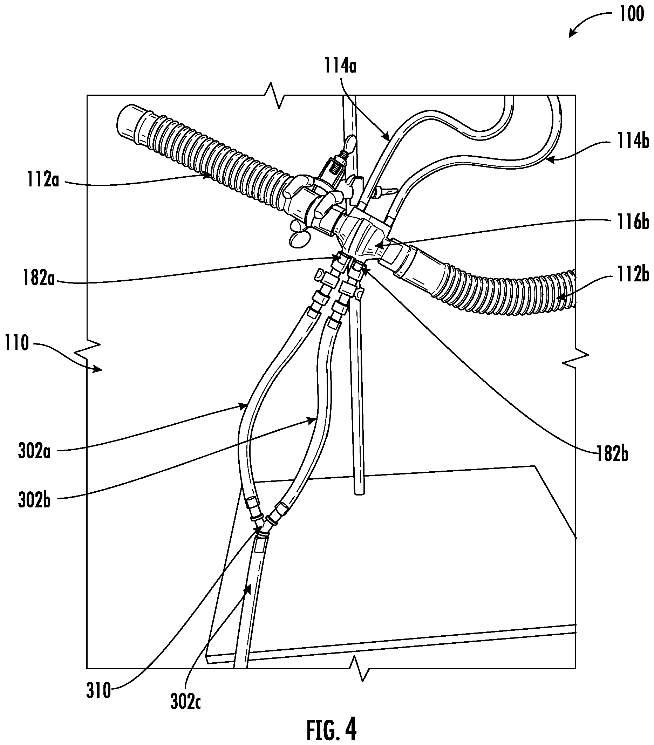

FIG. 4 schematically illustrates a portion of a breathing system 100 showing a suction tube assembly 190 in accordance with at least one example embodiment of the present disclosure. In some embodiments, and as shown in FIG. 4, the opposing ends of the first suction tube 302a and the second suction tube 302b may be coupled to a splitter such as a "Y" fitting 310. For example, as shown in FIG. 4, an end of a third suction tube 302c of the suction tube assembly 190 may be coupled to the tail portion tail portion of a "Y" fitting 310. The opposing end of the third suction tube 302c may be configured for being coupled to the suction device.

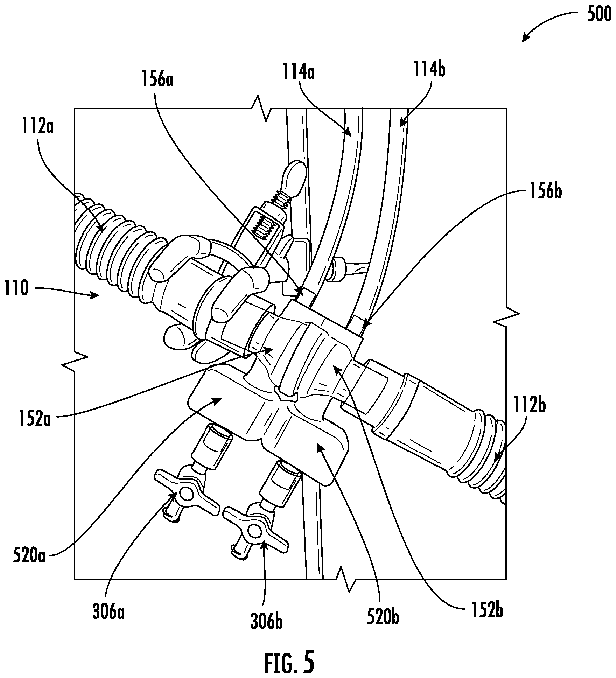

FIG. 5 schematically illustrates a portion of a breathing system 100 comprising a flow sensor 500 having one or more reservoirs in accordance with at least one example embodiment of the present disclosure. The flow sensor 500 may comprise several of the same components that have been described above in connection to the flow sensor 150. Therefore, a repeated description of the similar component are omitted.

In various embodiments, the flow sensor 150 includes at least one reservoir configured for receiving mucus, condensation, and/or other contamination. As shown in FIG. 5, the housing 152 may comprise a first reservoir 520a and a second reservoir 520b. The first reservoir 520a may be disposed opposite the first coupling member 156a and the second reservoir 520b may be disposed opposite the second coupling member 156b. As shown in FIG. 5, the third coupling member 182a and the fourth coupling member 182b may extend from the first reservoir 520a and the second reservoir 520b respectively in a direction opposite the direction in which the first coupling member 156a and the second coupling member 156b extend from the body 158a and the body 158b, respectively.

As shown in FIG. 5. in some embodiments, the third coupling member 182a may extend perpendicular from the first reservoir 520a and the fourth coupling member 182b may extend perpendicular from the second reservoir 520b. It would be appreciated that in some embodiments, the third coupling member 182a may extend at an angle that is not perpendicular with respect to the body 158a (e.g., first body portion 160a thereof) and the fourth coupling member 182b may extend at an angle that is not perpendicular with respect to the body 158b (e.g., first body portion 162a thereof). For example, each of the third coupling member 182a and fourth coupling member 182b may extend from the first reservoir 520a and the second reservoir 520b respectively, such that the third coupling member 182a extends away with respect to the vertical axis 102 defined by the housing 152 and the fourth coupling member 182b extends away with respect to the vertical axis 102. As another example, each of the third coupling member 182a and fourth coupling member 182b may extend from the first reservoir 520a and the second reservoir 520b respectively, such that the third coupling member 182a extends towards with respect to the vertical axis 102 defined by the housing 152 and the fourth coupling member 182b extends towards with respect to the vertical axis 102.

Each of the third coupling member 182a and the fourth coupling member 182b may define an opening therethrough (e.g., channel), such that contamination within the flow sensor 150 include contamination collected in the first reservoir 520a and the second reservoir 520b may be transmitted out of the first reservoir 520a and the second reservoir 520b via the third coupling member 182a and the fourth coupling member 182b respectively.

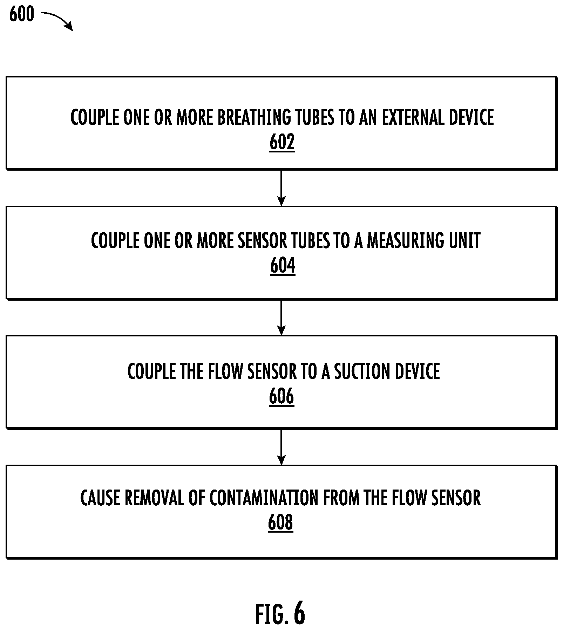

FIG. 6 illustrates a flowchart depicting operations of an example process for removing contamination from a flow sensor integrated within a breathing circuit in accordance with at least one example embodiment of the present disclosure. Specifically, FIG. 6 depicts an example process 600. Although the example processes depicts a particular sequence of operations, the sequence may be altered without departing from the scope of the present disclosure. For example, some of the operations depicted may be performed in parallel or in a different sequence that does not materially affect the function of the processes.

The blocks indicate operations of each process. Such operations may be performed in any of a number of ways, including, without limitation, in the order and manner as depicted and described herein. In some embodiments, one or more blocks of any of the processes described herein occur in-between one or more blocks of another process, before one or more blocks of another process, in parallel with one or more blocks of another process, and/or as a sub-process of a second process. Additionally or alternatively, any of the processes in various embodiments include some or all operational steps described and/or depicted, including one or more optional blocks in some embodiments. With regard to the flowcharts illustrated herein, one or more of the depicted block(s) in some embodiments is/are optional in some, or all, embodiments of the disclosure. Optional blocks are depicted with broken (or "dashed") lines. Similarly, it should be appreciated that one or more of the operations of each flowchart may be combinable, replaceable, and/or otherwise altered as described herein.

According to some examples, the method includes coupling one or more breathing tubes of the breathing circuit to one or more external devices at block 602. The at least one breathing tube may be coupled to the one or more external devices via one or more connectors and/or adapters. In some embodiments, one or more breathing tubes are connected to a ventilator device. For example, an end of the at least one breathing tube may be coupled to the ventilator device, wherein the opposing end of the breathing tube is coupled to the flow sensor integrated within the breathing circuit. The flow sensor may be disposed between a pair of breathing tubes. For example, a first end of the flow sensor may be coupled to a first end of first breathing tube and a second end of the flow sensor may be coupled to a first end of a second breathing tube, where a second end of the first breathing tube may be coupled to a ventilator device, and a second end of the second breathing tube may be configured for interaction with a user or coupled to other components configured for interaction with the user. For example, a portion of the second breathing tube may be configured for being inserted into a user's mouth.

According to some examples, the method includes coupling one or more sensor tubes to a measuring unit at block 604. In some embodiments, the measuring unit comprising one or more differential pressure measurement devices. For example, the measuring unit may comprise differential pressure measurement gauges. The one or more sensors may be coupled to the measuring unit via one or more connectors. In some embodiments, the measuring unit is embodied within the ventilator device. In such some examples, the one or more sensor tubes may be coupled to the ventilator device via one or more connectors.

According to some examples, the method includes coupling the flow sensor to a suction device at block 606. For example, the flow sensor may be coupled to a suction tube assembly (e.g., comprising one or more suction tubes) at one end of the suction tube assembly, where the suction tube assembly is coupled to the suction device via an opposing end of the suction device. In some embodiments, the suction tube assembly is coupled to the flow sensor via one or more coupling members of the flow sensor. The one or more coupling members may each define an opening therethrough, such that a flow path is created between the flow sensor and the suction tube assembly.

The one or coupling members may be coupled to the suction tube assembly via one or more connectors. In some embodiments, a luer lock fitting connection may be leveraged to couple the flow sensor to suction tube assembly. In some embodiments, one or more valves are disposed between the flow sensor and the one or more suction valves. The one or more suction valves may be configured to regulate opening, closing, and/or partial obstruction of one or more flow paths defined by the flow sensor and the suction tube assembly.

According to some examples, the method includes causing removal of contamination from the flow sensor via suction at block 608. In some embodiments, causing removal of contamination from the flow sensor comprises opening a valve positioned between the flow sensor and the suction tube assembly coupled to the suction device. In some embodiments, causing removal of contamination from the flow sensor comprises switching the suction device from an OFF state to an ON state (e.g., operation state of the suction device).

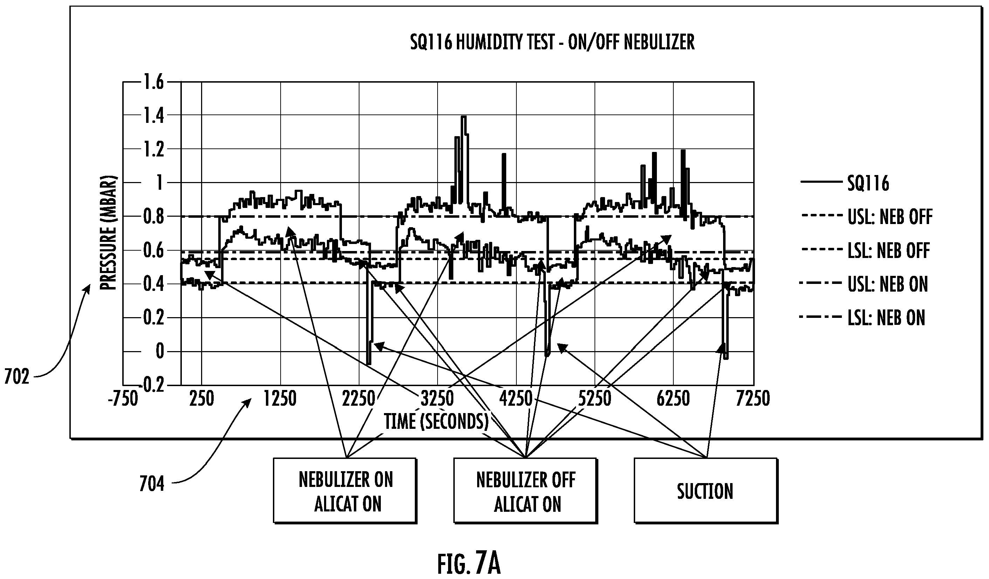

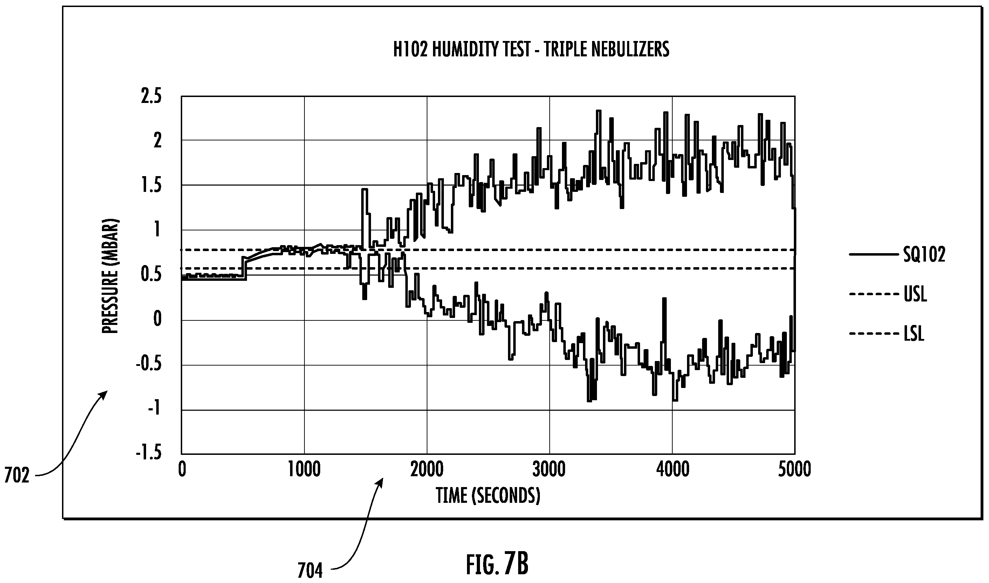

FIGS. 7a and 7b depict results of an example experimental simulation conducted to verify the effectiveness of embodiments as described herein. Specifically, FIG 7a depicts results of an example simulation conducted using a breathing system in accordance with at least one embodiment described herein. FIG. 7b depicts results of an example simulation conducted using a breathing circuit configuration without contamination removal as described herein. As shown in FIGS. 7a and 7b, the simulation result comprise a plot, wherein the y-axis represents pressure measurement 702, and the x-axis represents time 704.

As shown in FIG. 7a, the contamination removal configuration of the simulated example embodiment brings the flow sensor back within the example specification following contamination (e.g., by humidity) of the flow sensor as compared to the FIG. 7b (without suction). As shown in FIG. 7b, the flow sensor failed at a fast rate. As described above, the ability to suction in accordance with at least one embodiment of the present disclosure increases the time to failure and reduces medical provider intervention.

### Conclusion

Many modifications and other embodiments of the disclosure set forth herein will come to mind to one skilled in the art to which this disclosure pertains having the benefit of the teachings presented in the foregoing description and the associated drawings. Therefore, it is to be understood that the embodiments are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense.

While this specification contains many specific implementation details, these should not be construed as limitations on the scope of any disclosures or of what may be claimed, but rather as descriptions of features specific to particular embodiments of particular disclosures. Certain features that are described herein in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system components in the embodiments described above should not be understood as requiring such separation in all embodiments, and it should be understood that the described program components and systems can generally be integrated together in a single software product or packaged into multiple software products.

Thus, particular embodiments of the subject matter have been described. Other embodiments are within the scope of the following claims. In some cases, the actions recited in the claims can be performed in a different order and still achieve desirable results. In addition, the processes depicted in the accompanying figures do not necessarily require the particular order shown, or sequential order, to achieve desirable results. In certain implementations, multitasking and parallel processing may be advantageous.

## Claims

1. A breathing system, comprising:
a breathing circuit comprising a plurality of breathing tubes;
a flow sensor coupled to the breathing circuit, the flow sensor comprising a housing defining a flow channel and having a plurality of coupling members, wherein each coupling member of the plurality of coupling members defines an opening therethrough; and
a suction tube assembly, wherein:
a first set of coupling members of the plurality of coupling members is coupled to an end of the suction tube assembly via one or more connectors, and
one or more valves are disposed between the first set of coupling members and the suction tube assembly.

2. The breathing system of claim 1, wherein the housing comprises a first end coupled to a first breathing tube of the plurality of breathing tubes and an opposing second end coupled to a second breathing tube of the plurality of breathing tubes.

3. The breathing system of claim 1, wherein the one or more connectors comprise one or more secure leak-proof connectors.

4. The breathing system of claim 1, wherein the one or more valves are configured for regulating removal of contamination within the flow sensor via the suction tube assembly.

5. The breathing system of claim 1, wherein the housing comprises a first housing and a second housing, wherein a first coupling member of the first set of coupling members extends from the first housing, and a second coupling member of the first set of coupling members extends from the second housing.

6. The breathing system of claim 5, wherein each of the first coupling member and the second coupling member comprises a luer lock fitting.

7. The breathing system of claim 6, wherein the first coupling member is coupled to a first suction tube of the suction tube assembly, such that a first suction pathway is defined, and the second coupling member is coupled to a second suction tube of the suction tube assembly, such that a second suction pathway is defined.

8. The breathing system of claim 7, wherein a first valve of the one or more valves is disposed between the first coupling member and the first suction tube, and a second valve of the one or more valves is disposed between the second coupling member and the second suction tube.

9. The breathing system of claim 8, wherein an end of a third suction tube of the suction tube assembly is coupled to the first suction tube and the second suction tube via a connector.

10. The breathing system of claim 1, wherein an opposing end of the suction tube assembly is configured for being coupled to a suction device.

11. A breathing system, comprising:
a breathing circuit comprising a plurality of breathing tubes;
a flow sensor coupled to the breathing circuit, the flow sensor comprising a housing defining a flow channel and having at least one reservoir and a plurality of coupling members, wherein each coupling member of the plurality of coupling members defines an opening therethrough; and
a suction tube assembly, wherein:
a first set of coupling members of the plurality of coupling members is coupled to an end of the suction tube assembly via one or more connectors, and
one or more valves are disposed between the first set of coupling members and the suction tube assembly.

12. The breathing system of claim 11, wherein the housing comprises a first end coupled to a first breathing tube of the plurality of breathing tubes and an opposing second end coupled to a second breathing tube of the plurality of breathing tubes.

13. The breathing system of claim 11, wherein the one or more connectors comprise one or more secure leak-proof connectors, wherein the one or more valves are configured for regulating removal of contamination within the flow sensor via the suction tube assembly, and wherein the housing comprises a first housing having a first reservoir and a second housing having a second reservoir.

14. The breathing system of claim 13, wherein a first coupling member of the first set of coupling members extends from the first reservoir, and a second coupling member of the first set of coupling members extends from the second reservoir.

15. The breathing system of claim 14, wherein each of the first coupling member and the second coupling member comprises a luer lock fitting.
